# EUROPEAN PATENT APPLICATION

(11) **EP 2 921 513 A1**
(43) Date of publication of application: **23.09.2015**
(21) Application number: 13852931.8
(22) Date of filing: 30.10.2013
(51) Int. Cl.: C08G 59/20, C07D 491/044, C08G 59/02

(54) **EPOXY COMPOUND, PRODUCTION METHOD THEREFOR, AND CURABLE EPOXY RESIN COMPOSITION**

(30) Priority: 09.11.2012 JP 2012247380; 30.01.2013 JP 2013015642
(71) Applicant: Daicel Corporation, Osaka-shi, Osaka 530-0001 (JP)
(72) Inventor: INOUE, Hiroko, Ohtake-shi Hiroshima 739-0695 (JP)
(74) Representative: Grünecker Patent- und Rechtsanwälte PartG mbB
(86) International application number: PCT/JP2013/079399
(87) International publication number: WO 2014/073429

(57) **Abstract**

An object of the present invention is to provide a novel epoxy compound capable of forming a highly heat-resistant cured product. The epoxy compound of the present invention is a compound represented by the following formula (1): 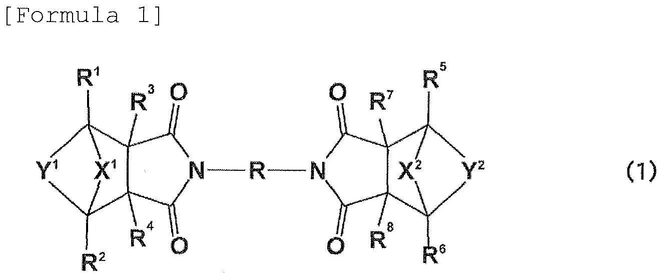 wherein X¹ and X² are the same or different and represent an alkylene group, an oxygen atom, a sulfur atom, or no bond; R represents a bivalent group having one or more atoms; R¹ to R⁸ are the same or different and represent a hydrogen atom, an alkenyl group which may have a substituent, an epoxidized alkenyl group which may have a substituent, or an alkyl group which may have a substituent; and Y¹ and Y² are the same or different and are a bivalent group represented by the following formula (a) : 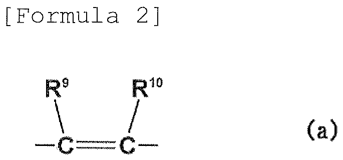 or a bivalent group represented by the following formula (b) : 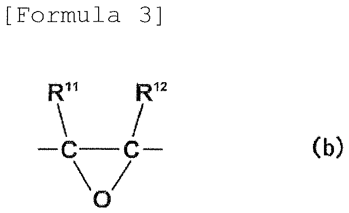 provided that least one of R¹ to R¹² in formula (1) is an alkenyl group which may have a substituent, an epoxidized alkenyl group which may have a substituent, or an alkyl group which may have a substituent. Besides, when both Y¹ and Y² in formula (1) are a bivalent group represented by formula (a), at least one of R¹ to R¹⁰ is an epoxidized alkenyl group which may have a substituent.

## Description

### Technical Field

The present invention relates to a novel epoxy compound, a production method therefor, and a curable epoxy resin composition (a curable composition) containing the epoxy compound. The present application is based upon and claims the benefit of priority of Japanese Patent Application No. 2012-247380, filed in Japan on November 9, 2012, and Japanese Patent Application No. 2013-015642, filed in Japan on January 30, 2013, the entire contents of which are incorporated herein

### Background Art

Epoxy resins have excellent thermal, mechanical and electrical characteristics, and hence are used in various applications including adhesives, coating materials, electrical and electronic materials, and constructional materials. The applications of the epoxy resins have been expanded year by year, and under this situation, further enhancement of the functionality of the epoxy resins is required, and there is an increasing demand particularly for the heat resistance in recent years.

Among the epoxy resins, an alicyclic epoxy resin (an epoxy resin having an alicyclic structure) in particular is known as a material excellent in transparency and heat resistance (for example, Patent Literature 1). Even the alicyclic epoxy resin thus regarded to have high heat resistance has, however, a problem of still insufficient heat resistance particularly when used in applications where the resin is exposed to a high temperature. Therefore, under the present circumstances, an epoxy resin further excellent in the heat resistance is desired to be developed.

### Citation List

### Patent Literature

Patent Literature 1: Japanese Patent Laid-Open No. 5-160299

### Summary of Invention

### Technical Problem

Accordingly, an object of the present invention is to provide a novel epoxy compound capable of forming a highly heat-resistant cured product (cured resin product).

Another object of the present invention is to provide a curable epoxy resin composition capable of forming a highly heat-resistant cured product (and further preferably, also excellent in handleability).

### Solution to Problem

The present inventor has found, as a result of earnest studies made for overcoming the aforementioned problem, that an epoxy compound of a specific structure having both an epoxy group and an imide skeleton can form a highly heat-resistant cured product, resulting in accomplishing the present invention.

Specifically, the present invention provides an epoxy compound represented by the following formula (1): 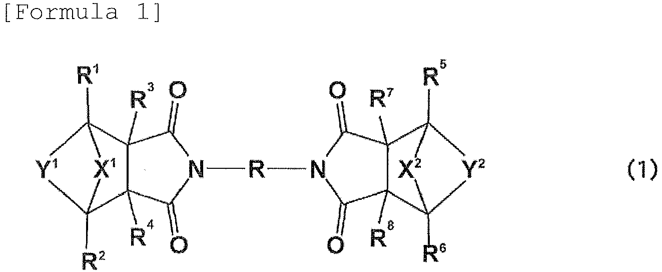 wherein X¹ and X² are the same or different and represent an alkylene group, an oxygen atom, a sulfur atom, or no bond; R represents a bivalent group having one or more atoms; R¹ to R⁸ are the same or different and represent a hydrogen atom, an alkenyl group which may have a substituent, an epoxidized alkenyl group which may have a substituent, or an alkyl group which may have a substituent; and Y¹ and Y² are the same or different and represent a bivalent group represented by the following formula (a): 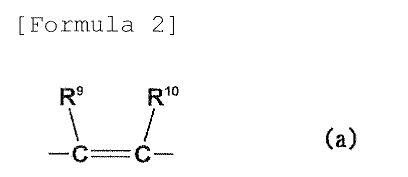 or a bivalent group represented by the following formula (b) :

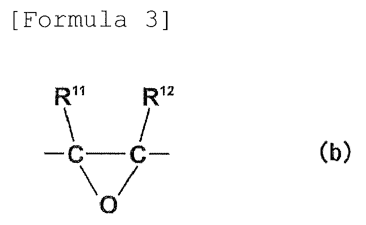

R⁹ and R¹⁰ in formula (a) are the same or different and represent a hydrogen atom, an alkenyl group which may have a substituent, an epoxidized alkenyl group which may have a substituent, or an alkyl group which may have a substituent; and R¹¹ and R¹² in formula (b) are the same or different and represent a hydrogen atom, an alkenyl group which may have a substituent, an epoxidized alkenyl group which may have a substituent, or an alkyl group which may have a substituent; provided that at least one of R¹ to R¹² in formula (1) is an alkenyl group which may have a substituent, an epoxidized alkenyl group which may have a substituent, or an alkyl group which may have a substituent; and when both Y¹ and Y² in formula (1) area bivalent group represented by formula (a), at least one of R¹ to R¹⁰ is an epoxidized alkenyl group which may have a substituent.

Besides, the present invention provides a production method for an epoxy compound, in which a compound represented by the following formula (2): 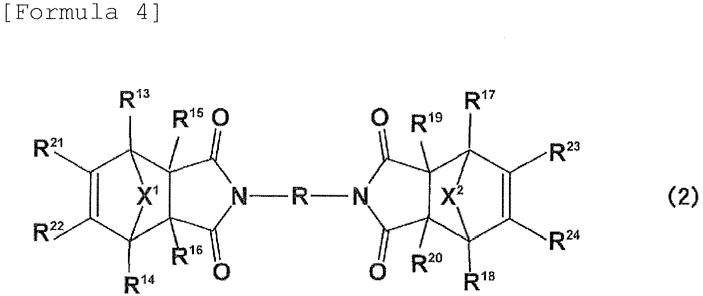 wherein X¹ and X² are the same or different and represent an alkylene group, an oxygen atom, a sulfur atom or no bond; R represents a bivalent group having one or more atoms; R¹³ to R²⁴ are the same or different and represent a hydrogen atom, an alkenyl group which may have a substituent, or an alkyl group which may have a substituent; provided that at least one of R¹³ to R²⁴ in formula (2) is an alkenyl group which may have a substituent or an alkyl group which may have a substituent; is reacted with an oxidant to produce an epoxy compound represented by the following formula (1): 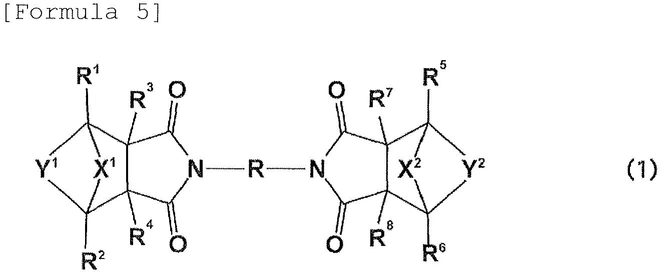 wherein X¹, X² and R are the same as defined above; R¹ to R⁸ are the same or different and represent a hydrogen atom, an alkenyl group which may have a substituent, an epoxidized alkenyl group which may have a substituent, or an alkyl group which may have a substituent; Y¹ and Y² are the same or different and represent a bivalent group represented by the following formula (a): 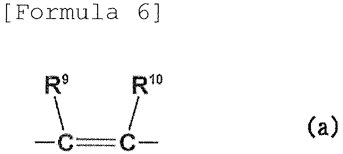 or a bivalent group represented by the following formula (b) : 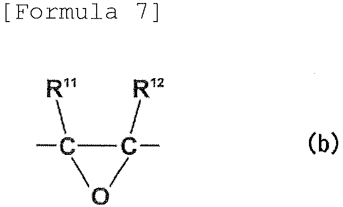 R⁹ and R¹⁰ in formula (a) are the same or different and represent a hydrogen atom, an alkenyl group which may have a substituent, an epoxidized alkenyl group which may have a substituent, or an alkyl group which may have a substituent; and R¹¹ and R¹² in formula (b) are the same or different and represent a hydrogen atom, an alkenyl group which may have a substituent, an epoxidized alkenyl group which may have a substituent, or an alkyl group which may have a substituent; provided that at least one of R¹ to R¹² in formula (1) is an alkenyl group which may have a substituent, an epoxidized alkenyl group which may have a substituent, or an alkyl group which may have a substituent; and when both Y¹ and Y² in formula (1) are a bivalent group represented by formula (a), at least one of R¹ to R¹⁰ is an epoxidized alkenyl group which may have a substituent.

Furthermore, the present invention provides a curable epoxy resin composition comprising the above-described epoxy compound.

Besides, the present invention provides the above-described curable epoxy resin composition, which is in a liquid form at 25°C.

Besides, the present invention provides the above-described curable epoxy resin composition, further containing an alicyclic epoxy compound that is in a liquid form at 25°C.

Additionally, the present invention provides a cured product obtained by curing the above-described curable epoxy resin composition.

Specifically, the present invention relates to the following:
(1) An epoxy compound represented by the following formula (1): 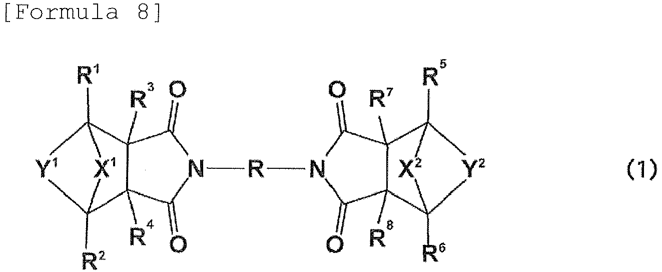 wherein X¹ and X² are the same or different and represent an alkylene group, an oxygen atom, a sulfur atom, or no bond; R represents a bivalent group having one or more atoms; R¹ to R⁸ are the same or different and represent a hydrogen atom, an alkenyl group which may have a substituent, an epoxidized alkenyl group which may have a substituent, or an alkyl group which may have a substituent; and Y¹ and Y² are the same or different and represent a bivalent group represented by the following formula (a): 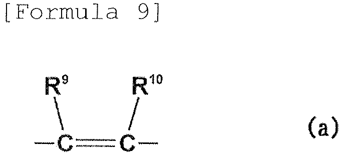 or a bivalent group represented by the following formula (b) : 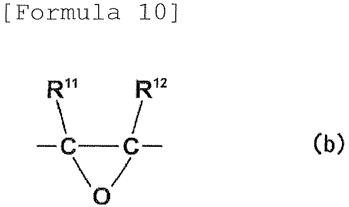 R⁹ and R¹⁰ in formula (a) are the same or different and represent a hydrogen atom, an alkenyl group which may have a substituent, an epoxidized alkenyl group which may have a substituent, or an alkyl group which may have a substituent; and R¹¹ and R¹² in formula (b) are the same or different and represent a hydrogen atom, an alkenyl group which may have a substituent, an epoxidized alkenyl group which may have a substituent, or an alkyl group which may have a substituent; provided that at least one of R¹ to R¹² in formula (1) is an alkenyl group which may have a substituent, an epoxidized alkenyl group which may have a substituent, or an alkyl group which may have a substituent; and when both Y¹ and Y² in formula (1) are a bivalent group represented by formula (a), at least one of R¹ to R¹⁰ is an epoxidized alkenyl group which may have a substituent.
(2) The epoxy compound according to (1), wherein the epoxy compound according to (1) is selected from the group consisting of compounds represented by the following formulas (1'): 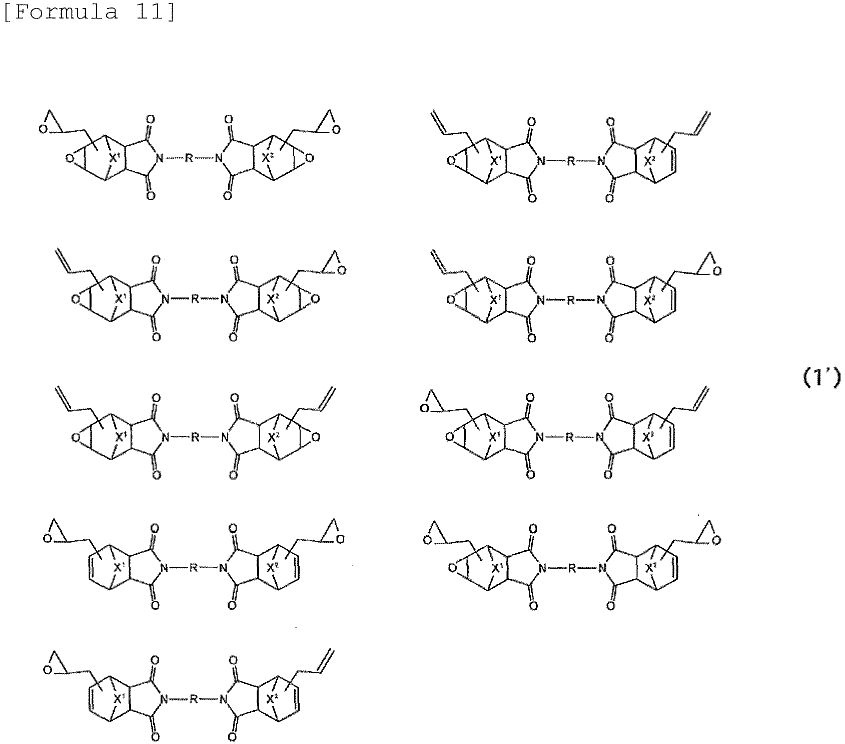 wherein X¹, X² and R have the same meanings as X¹, X² and R in formula (1), respectively.
(3) The epoxy compound according to (1) or (2), in which X¹ and X² are a methylene group.
(4) The epoxy compound according to any one of (1) to (3), in which R is a bivalent hydrocarbon group.
(5) The epoxy compound according to any one of (1) to (4), in which R is an alkylene group, a bivalent group represented by the following formula (i), a bivalent group represented by the following formula (ii), a bivalent group represented by the following formula (iii), or a bivalent group represented by the following formula (iv) : 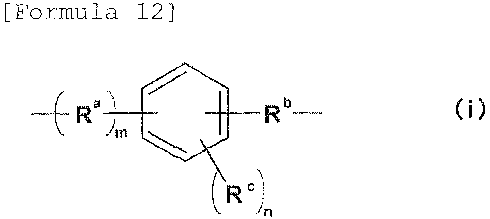 wherein R^{a} and R^{b} are the same or different and represent an alkylene group having 1 to 4 carbon atoms or a cycloalkylene group having 5 to 8 carbon atoms; R^{c} represents an alkyl group, a halogen atom, a hydroxy group, a carboxy group, an alkoxy group, an alkenyloxy group, an aryloxy group, an aralkyloxy group, an acyloxy group, a mercapto group, an alkylthio group, an alkenylthio group, an arylthio group, an aralkylthio group, an alkoxycarbonyl group, an aryloxycarbonyl group, an aralkyloxycarbonyl group, an amino group, a nitro group, a mono- or di-alkylamino group, an acylamino group, an epoxy group, a glycidyl group, an acyl group, a cyano group, an isocyanate group, an isothiocyanate group, a carbamoyl group, or a sulfo group; m is 0 or 1; and n is an integer of 0 to 4; 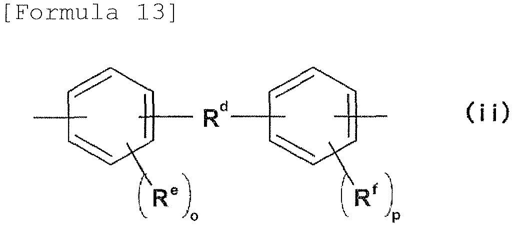 wherein R^{d} represents an alkylene group having 1 to 4 carbon atoms, or a cycloalkylene group having 5 to 8 carbon atoms; R^{e} and R^{f} are the same or different and represent an alkyl group, a halogen atom, a hydroxy group, a carboxy group, an alkoxy group, an alkenyloxy group, an aryloxy group, an aralkyloxy group, an acyloxy group, a mercapto group, an alkylthio group, an alkenylthio group, an arylthio group, an aralkylthio group, an alkoxycarbonyl group, an aryloxycarbonyl group, an aralkyloxycarbonyl group, an amino group, a nitro group, a mono- or di-alkylamino group, an acylamino group, an epoxy group, a glycidyl group, an acyl group, a cyano group, an isocyanate group, an isothiocyanate group, a carbamoyl group, or a sulfo group; and o and p are the same or different and are an integer of 0 to 4; 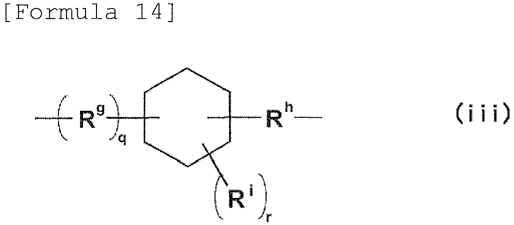 wherein R^{g} and R^{h} are the same or different and represent an alkylene group having 1 to 4 carbon atoms, or an arylene group having 6 to 12 carbon atoms; Rⁱ represents an alkyl group, a halogen atom, a hydroxy group, a carboxy group, an alkoxy group, an alkenyloxy group, an aryloxy group, an aralkyloxy group, an acyloxy group, a mercapto group, an alkylthio group, an alkenylthio group, an arylthio group, an aralkylthio group, an alkoxycarbonyl group, an aryloxycarbonyl group, an aralkyloxycarbonyl group, an amino group, a nitro group, a mono- or di-alkylamino group, an acylamino group, an epoxy group, a glycidyl group, an acyl group, a cyano group, an isocyanate group, an isothiocyanate group, a carbamoyl group, or a sulfo group; q represents 0 or 1; and r is an integer of 0 to 10; 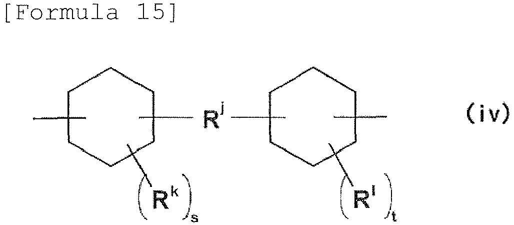 wherein R^{j} represents an alkylene group having 1 to 4 carbon atoms, or an arylene group having 6 to 12 carbon atoms; R^{k} and R^{l} are the same or different and represent an alkyl group, a halogen atom, a hydroxy group, a carboxy group, an alkoxy group, an alkenyloxy group, an aryloxy group, an aralkyloxy group, an acyloxy group, a mercapto group, an alkylthio group, an alkenylthio group, an arylthio group, an aralkylthio group, an alkoxycarbonyl group, an aryloxycarbonyl group, an aralkyloxycarbonyl group, an amino group, a nitro group, a mono- or di-alkylamino group, an acylamino group, an epoxy group, a glycidyl group, an acyl group, a cyano group, an isocyanate group, an isothiocyanate group, a carbamoyl group, or a sulfo group; and s and t are the same or different and are an integer of 0 to 10.
(6) The epoxy compound according to (5), in which the bivalent group represented by formula (iii) above is a group represented by the following formula (iii'): 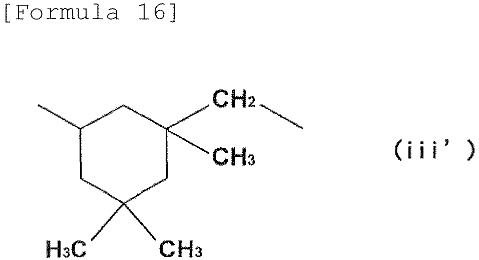
(7) A production method for an epoxy compound, in which a compound represented by the following formula (2): 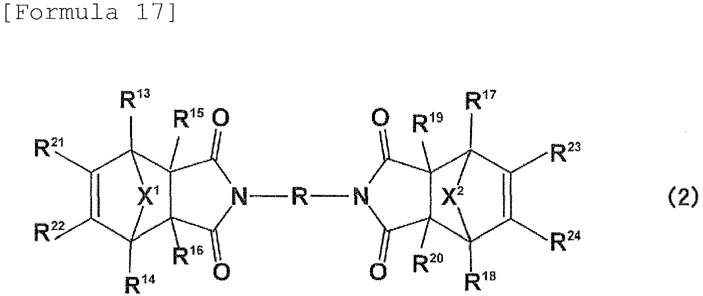 wherein X¹ and X² are the same or different and represent an alkylene group, an oxygen atom, a sulfur atom or no bond; R represents a bivalent group having one or more atoms; and R¹³ to R²⁴ are the same or different and represent a hydrogen atom, an alkenyl group which may have a substituent, or an alkyl group which may have a substituent; provided that at least one of R¹³ to R²⁴ in formula (2) is an alkenyl group which may have a substituent or an alkyl group which may have a substituent,
   is reacted with an oxidant to produce an epoxy compound represented by the following formula (1): 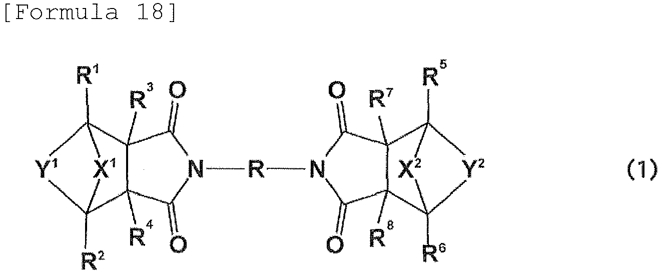 wherein X¹, X² and R are the same as those defined above, R¹ to R⁸ are the same or different and represent a hydrogen atom, an alkenyl group which may have a substituent, an epoxidized alkenyl group which may have a substituent, or an alkyl group which may have a substituent; and Y¹ and Y² are the same or different and represent a bivalent group represented by the following formula (a): 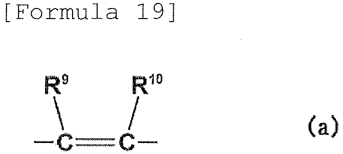 or a bivalent group represented by the following formula (b) : 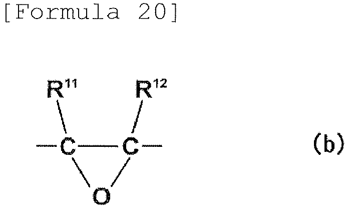 R⁹ and R¹⁰ in formula (a) are the same or different and represent a hydrogen atom, an alkenyl group which may have a substituent, an epoxidized alkenyl group which may have a substituent, or an alkyl group which may have a substituent; and R¹¹ and R¹² in formula (b) are the same or different and represent a hydrogen atom, an alkenyl group which may have a substituent, an epoxidized alkenyl group which may have a substituent, or an alkyl group which may have a substituent; provided that at least one of R¹ to R¹² in formula (1) is an alkenyl group which may have a substituent, an epoxidized alkenyl group which may have a substituent, or an alkyl group which may have a substituent; and when both Y¹ and Y² in formula (1) are a bivalent group represented by formula (a), at least one of R¹ to R¹⁰ is an epoxidized alkenyl group which may have a substituent.
(8) A curable epoxy resin composition containing the epoxy compound according to any one of (1) to (6).
(9) The curable epoxy resin composition according to (8), in which the content of the epoxy compound according to any one of (1) to (6) is 1 to 99% by weight based on the total amount (100% by weight) of epoxy compounds contained in the curable epoxy resin composition.
(10) The curable epoxy resin composition according to (8) or (9), which is in a liquid form at 25°C.
(11) The curable epoxy resin composition according to any one of (8) to (10), which has a viscosity at 25°C of not more than 1,000,000 mPa·s.
(12) The curable epoxy resin composition according to any one of (8) to (11), further containing an alicyclic epoxy compound that is in a liquid form at 25°C.
(13) The curable epoxy resin composition according to (12), in which the alicyclic epoxy compound that is in a liquid form at 25°C is a compound represented by the following formula (I): 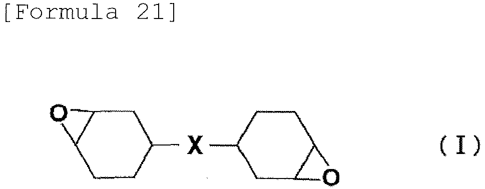 wherein X represents a single bond or a linking group.
(14) The curable epoxy resin composition according to (12) or (13), in which the content of the alicyclic epoxy compound that is in a liquid form at 25°C is 1 to 99% by weight based on the total amount (100% by weight) of epoxy compounds contained in the curable epoxy resin composition.
(15) A cured product obtained by curing the curable epoxy resin composition according to any one of (8) to (14).

### Advantageous Effects of Invention

An epoxy compound of the present invention and a curable epoxy resin composition of the present invention containing the epoxy compound have the aforementioned constitutions, and therefore, they can form a highly heat-resistant cured product. The epoxy compound of the present invention is also excellent in handleability particularly when it has excellent solubility in another epoxy compound. Besides, particularly when the curable epoxy resin composition of the present invention contains an alicyclic epoxy compound that is in a liquid form at room temperature or when the curable epoxy resin composition is in a liquid form at room temperature, it is excellent in the handleability.

### Brief Description of Drawings

[Figure 1] Figure 1 is a ¹H-NMR spectrum chart of an epoxy compound obtained in Example 1 (an epoxy compound according to the present invention).
[Figure 2] Figure 2 is a ¹³C-NMR spectrum chart of the epoxy compound obtained in Example 1 (the epoxy compound according to the present invention).
[Figure 3] Figure 3 is a UV chromatogram, obtained by LC-MS measurement, of an epoxy compound obtained in Example 2 (an epoxy compound according to the present invention).
[Figure 4] Figure 4 is a total ion chromatogram, obtained by the LC-MS measurement, of the epoxy compound obtained in Example 2.
[Figure 5] Figure 5(a) illustrates a mass spectrum of a peak at the retention time of 15 to 20 minutes in the LC-MS chromatogram of the epoxy compound obtained in Example 2, and Figure 5(b) is a mass chromatogram of a substance having m/z of 595.
[Figure 6] Figure 6(a) illustrates a mass spectrum of a peak at the retention time of 20 to 22 minutes in the LC-MS chromatogram of the epoxy compound obtained in Example 2, and Figure 6(b) is a mass chromatogram of a substance having m/z of 541.
[Figure 7] Figure 7(a) illustrates a mass spectrum of a peak at the retention time of 23 to 26 minutes in the LC-MS chromatogram of the epoxy compound obtained in Example 2, and Figure 7(b) is a mass chromatogram of a substance having m/z of 525.

### Description of Embodiments

### [Epoxy Compound]

An epoxy compound of the present invention is a compound represented by the following formula (1):

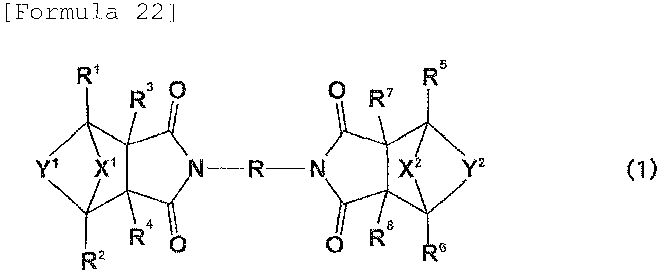

In formula (1) above, X¹ and X² are the same or different and represent an alkylene group, an oxygen atom, a sulfur atom or no bond. Examples of the alkylene group include straight or branched alkylene groups having 1 to 9 carbon atoms, such as a methylene group, a methyl methylene group, a dimethyl methylene group, an ethylene group, a propylene group, a trimethylene group, and a hexamethylene group. As X¹ and X², a methylene group is particularly preferred.

In formula (1), R represents a bivalent group (a linking group) having one or more atoms. Examples of the linking group include a bivalent hydrocarbon group.

Examples of the bivalent hydrocarbon group include a bivalent aliphatic hydrocarbon group, a bivalent alicyclic hydrocarbon group, and a bivalent aromatic hydrocarbon group. Examples of the bivalent aliphatic hydrocarbon group include straight or branched alkylene groups having 1 to 18 carbon atoms, such as a methylene group, a methylmethylene group, a dimethylmethylene group, an ethylene group, a propylene group, and a trimethylene group. Examples of the bivalent alicyclic hydrocarbon group include bivalent cycloalkylene groups (including cycloalkylidene groups), such as a 1,2-cyclopentylene group, a 1,3-cyclopentylene group, a cyclopentylidene group, a 1,2-cyclohexylene group, 1,3-cyclohexylene group, a 1,4-cyclohexylene group, and a cyclohexylidene group. Examples of the bivalent aromatic hydrocarbon group include arylene groups, such as a phenylene group, a tolylene group, a dimethylphenylene group and a naphthylene group. Incidentally, an aliphatic ring (an aliphatic hydrocarbon ring) of the bivalent alicyclic hydrocarbon group and an aromatic ring of the bivalent aromatic hydrocarbon group may have a substituent (a monovalent group), such as an alkyl group, a halogen atom, a hydroxy group, a carboxy group, an alkoxy group, an alkenyloxy group, an aryloxy group, an aralkyloxy group, an acyloxy group, a mercapto group, an alkylthio group, an alkenylthio group, an arylthio group, an aralkylthio group, an alkoxycarbonyl group, an aryloxycarbonyl group, an aralkyloxycarbonyl group, an amino group, a nitro group, a mono- or di-alkylamino group, an acylamino group, an epoxy group, a glycidyl group, an acyl group, a cyano group, an isocyanate group, an isothiocyanate group, a carbamoyl group, or a sulfo group.

Besides, other example of the bivalent hydrocarbon group includes a bivalent group formed by a direct bond of two or more of a bivalent aliphatic hydrocarbon group, a bivalent alicyclic hydrocarbon group and a bivalent aromatic hydrocarbon group (which group is sometimes designated as the "bonded hydrocarbon group"). Examples of the bonded hydrocarbon group include an alkylene-arylene group, an alkylene-arylene-alkylene group, a cycloalkylene-arylene-cycloalkylene group, a cycloalkylene-arylene-alkylene group, an alkylene-cycloalkylene group, a cycloalkylene-arylene group, an arylene-alkylene-arylene group, and a cycloalkylene-alkylene-cycloalkylene group.

More specifically, examples of the bivalent bonded hydrocarbon group include bivalent groups represented by the following formulas (i) to (iv):

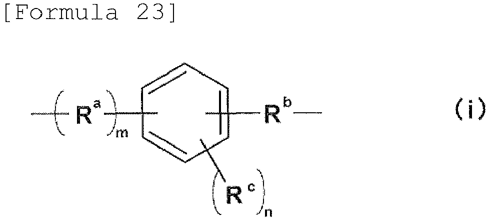

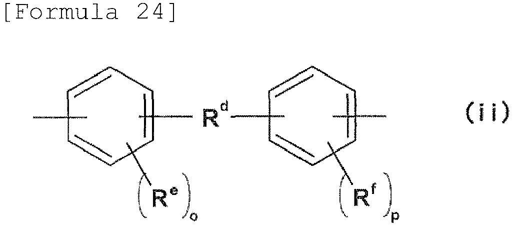

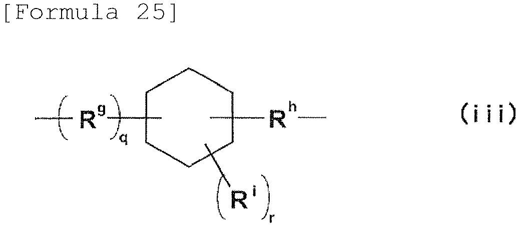

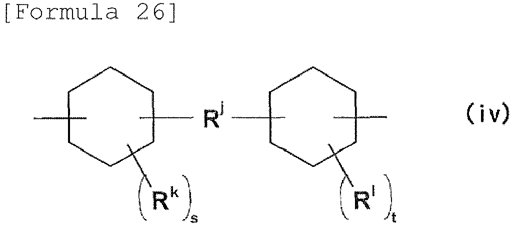

In formula (i) above, R^{a} and R^{b} are the same or different and represent an alkylene group having 1 to 4 carbon atoms or a cycloalkylene group having 5 to 8 carbon atoms. R^{c} represents a substituent (a monovalent group) on an aromatic ring represented in the formula, and examples include an alkyl group, a halogen atom, a hydroxy group, a carboxy group, an alkoxy group, an alkenyloxy group, an aryloxy group, an aralkyloxy group, an acyloxy group, a mercapto group, an alkylthio group,
an alkenylthio group, an arylthio group, an aralkylthio group, an alkoxycarbonyl group, an aryloxycarbonyl group, an aralkyloxycarbonyl group, an amino group, a nitro group, a mono- or di-alkylamino group, an acylamino group, an epoxy group, a glycidyl group, an acyl group, a cyano group, an isocyanate group, an isothiocyanate group, a carbamoyl group, and a sulfo group. Besides, m is 0 or 1. Furthermore, n represents the number of substituents on the aromatic ring represented in the formula, and is an integer of 0 to 4. Incidentally, if two or more R^{c} are contained, these R^{c} may be the same or different from each other.

More specifically, examples of the group represented by formula (i) above include: phenylene-alkylene groups, such as a phenylene-methylene group (in which phenylene may be any one of o-phenylene, m-phenylene and p-phenylene, which also applies to the following description), a phenylene-ethylene group, a phenylene-trimethylene group, a phenylene-tetramethylene group, a phenylene-butylidene group, a phenylene-s-butylidene group, a phenylene-1,2-dimethylethylene group, and a phenylene-1,1-dimethylethylene group; phenylene-cycloalkylene groups, such as a phenylene-1,2-cyclopentylene group, a phenylene-1,3-cyclopentylene group, a phenylene-1,4-cyclopentylene group, a phenylene-2-methyl-1,4-cyclopentylene group, a phenylene-2,3-dimethyl-1,4-cyclopentylene group, a phenylene-1,2-cyclohexylene group, a phenylene-1,4-cyclohexylene group, a phenylene-2-methyl-1,3-cyclohexylene group, a phenylene-3-methyl-1,4-cyclohexylene group, a phenylene-3-ethyl-1,4-cyclohexylene group, a phenylene-1,3-cycloheptylene group, a phenylene-3-methyl-1,4-cycloheptylene group, a phenylene-4-methyl-1,3-cycloheptylene group, a phenylene-1,3-cyclooctylene group, and a phenylene-1,4-cyclooctylene group; alkylene-phenylene-alkylene groups, such as an o-xylylene group, a m-xylylene group, a p-xylylene group, a methylene-phenylene-ethylene group, a methylene-phenylene-trimethylene group, a methylene-phenylene-1,2-dimethylethylene group, an ethylene-phenylene-butylidene group, an ethylene-phenylene-ethylene group, a butylidene-phenylene-butylidene group, and a trimethylene-phenylene-s-butylidene group; alkylene-phenylene-cycloalkylene groups, such as a methylene-phenylene-1,4-cyclopentylene group, an ethylene-phenylene-1,3-cyclopentylene group, and a trimethylene-phenylene-1,3-cyclooctylene group; and cycloalkylene-phenylene-cycloalkylene groups, such as a 1,4-cyclopentylene-phenylene-1,4-cyclopentylene group, a 1,3-cyclopentylene-phenylene-3-ethyl-1,4-cyclohexylene group, a 1,3-cyclooctylene-phenylene-1,3-cyclooctylene group, and a 1,4-cyclohexylene-phenylene-1,3-cyclooctylene group.

In formula (ii) above, R^{d} represents an alkylene group having 1 to 4 carbon atoms, or a cycloalkylene group having 5 to 8 carbon atoms. R^{e} and R^{f} represent a substituent (a monovalent group) on an aromatic ring represented in the formula and are the same or different, and examples include the same groups as those described above as the examples of R^{c} in formula (i) above. Besides, o and p represent the number of substituents on a corresponding aromatic ring represented in the formula, are the same or different, and are an integer of 0 to 4. Incidentally, when two or more R^{e} are contained, these R^{e} may be the same or different from each other. When two or more R^{f} are contained, these R^{f} may be the same or different from each other.

More specifically, examples of the group represented by formula (ii) above include: phenylene-alkylene-phenylene groups, such as a phenylene-methylene-phenylene group, a phenylene-ethylene-phenylene group, a phenylene-trimethylene-phenylene group, a phenylene-tetramethylene-phenylene group, and a phenylene-butylidene-phenylene group; and phenylene-cycloalkylene-phenylene groups, such as a phenylene-1,2-cyclopentylene-phenylene group, a phenylene-1,3-cyclopentylene-phenylene group, a phenylene-cyclopentylidene-phenylene group, a phenylene-1,2-cyclohexylene-phenylene group, a phenylene-1,3-cyclohexylene-phenylene group, a phenylene-1,4-cyclohexylene-phenylene group, a phenylene-cyclohexylidene-phenylene group, a phenylene-1,3-cyclooctylene-phenylene group, and a phenylene-1,4-cyclooctylene-phenylene group.

In formula (iii) above, R^{g} and R^{h} are the same or different and represent an alkylene group having 1 to 4 carbon atoms, or an arylene group having 6 to 12 carbon atoms. Rⁱ represents the substituents on a cyclohexane ring represented in the formula, and is the same or different, and examples include the same groups as those described above as examples of R^{c} in formula (i). Besides, q represents 0 or 1. Furthermore, r represents the number of substituents on the cyclohexane ring represented in the formula, is the same or different, and is an integer of 0 to 10. Incidentally, when two or more Rⁱ are contained, these Rⁱ may be the same or different from each other.

More specifically, examples of the group represented by formula (iii) above include: cyclohexylene-alkylene groups, such as a 1,2-cyclohexylene-methylene group, a 1,3-cyclohexylene-methylene group, a 1,4-cyclohexylene-methylene group, a cyclohexylidene-methylene group, a 1,2-cyclohexylene-ethylene group, a 1,3-cyclohexylene-ethylene group, a 1,4-cyclohexylene-ethylene group, a cyclohexylidene-ethylene group, and a methylene-1,5,5-trimethyl-1,3-cyclohexylene group (a bivalent group formed by removing two amino groups from isophorone diamine); cyclohexylene-arylene groups, such as a 1,2-cyclohexylene-phenylene group, a 1,3-cyclohexylene-phenylene group, and a 1,4-cyclohexylene-phenylene group; alkylene-cyclohexylene-alkylene groups, such as a methylene-1,2-cyclohexylene-methylene group, a methylene-1,3-cyclohexylene-methylene group, and a methylene-1,4-cyclohexylene-methylene group; alkylene-cyclohexylene-phenylene groups, such as a methylene-1,2-cyclohexylene-phenylene group, a methylene-1,3-cyclohexylene-phenylene group, and a methylene-1,4-cyclohexylene-phenylene group; and arylene-cyclohexylene-arylene groups, such as a phenylene-1,2-cyclohexylene-phenylene group, a phenylene-1,3-cyclohexylene-phenylene group, and a phenylene-1,4-cyclohexylene-phenylene group.

A specific example of the group represented by formula (iii) above includes a group represented by the following formulas (iii'):

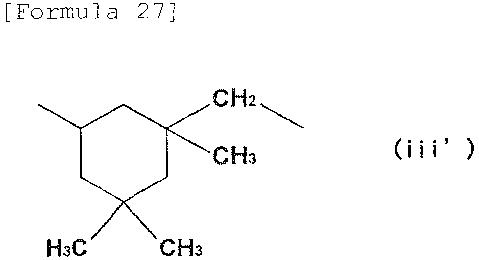

In formula (iv) above, R^{j} represents an alkylene group having 1 to 4 carbon atoms, or an arylene group having 6 to 12 carbon atoms. R^{k} and R^{l} are a substituent on a corresponding cyclohexane ring represented in the formula, and are the same or different, and examples include the same groups as those described above as the examples of R^{c} in formula (i) above. Besides, s and t represent the number of substituents on the cyclohexane ring represented in the formula, are the same or different, and are an integer of 0 to 10. Incidentally, if two or more R^{k} are contained, these R^{k} may be the same or different from each other. If two or more R^{l} are contained, these R^{l} may be the same or different from each other.

More specifically, examples of the group represented by formula (iv) above include: cyclohexylene-alkylene-cyclohexylene groups, such as a 1,2-cyclohexylene-methylene-1,2-cyclohexylene group, a 1,3-cyclohexylene-methylene-1,3-cyclohexylene group, a 1,4-cyclohexylene-methylene-1,4-cyclohexylene group, a 1,2-cyclohexylene-ethylene-1,2-cyclohexylene group, a 1,3-cyclohexylene-ethylene-1,3-cyclohexylene group, and a 1,4-cyclohexylene-ethylene-1,4-cyclohexylene group; and cyclohexylene-phenylene-cyclohexylene groups, such as a 1,2-cyclohexylene-phenylene-1,2-cyclohexylene group, a 1,3-cyclohexylene-phenylene-1,3-cyclohexylene group, and a 1,4-cyclohexylene-phenylene-1,4-cyclohexylene group.

Other examples of R (the linking group) in formula (1) above include a bivalent group formed by a bond of one or more bivalent hydrocarbon groups and one or more bivalent groups having a hetero atom, such as a carbonyl group, an ether bond, an ester bond, a carbonate group, or an amide group.

Among these examples, as R in formula (1), an alkylene group (a hexamethylene group in particular), a bivalent group represented by formula (i) (a xylylene group in particular), a bivalent group represented by formula (ii) (a phenylene-methylene-phenylene group in particular) and a bivalent group represented by formula (iii) are particularly preferred.

In formula (1) above, R¹ to R⁸ are the same or different and represent a hydrogen atom, an alkenyl group which may have a substituent, an epoxidized alkenyl group which may have a substituent, or an alkyl group which may have a substituent. Examples of the alkenyl group include a vinyl group, an allyl group (a 2-propenyl group), a methallyl group (a 2-methylallyl group), a 1-propenyl group, an isopropenyl group, a 1-butenyl group, and a 2-butenyl group. The epoxidized alkenyl group means the groups in which a carbon-carbon unsaturated double bond in the above-described alkenyl group is epoxidized, and examples include an epoxidized allyl group (a glycidyl group) and an epoxidized methallyl group (a 2-methylglycidyl group). Examples of the alkyl group include alkyl groups having 1 to 6 carbon atoms, such as a methyl group, an ethyl group, a propyl group, an isopropyl group, a butyl group, a s-butyl group, an isobutyl group, a t-butyl group, a pentyl group, and a hexyl group.

Examples of the substituent that the alkenyl group, the epoxidized alkenyl group and the alkyl group may have include substituents having 0 to 20 carbon atoms (more preferably, 0 to 10 carbon atoms). Examples of the substituent include: a halogen atom such as a fluorine atom, a chlorine atom, a bromine atom, or an iodine atom; a hydroxy group; an alkoxy group (preferably a C₁₋₆ alkoxy group, and more preferably a C₁₋₄ alkoxy group) such as a methoxy group, an ethoxy group, a propoxy group, an isopropyloxy group, a butoxy group, or an isobutyloxy group; an alkenyloxy group (preferably a C₂₋₆ alkenyloxy group, and more preferably a C₂₋₄ alkenyloxy group) such as an allyloxy group; an aryloxy group (preferably a C₆₋₁₄ aryloxy group) which may have a substituent of a C₁₋₄ alkyl group, a C₂₋₄ alkenyl group, a halogen atom, a C₁₋₄ alkoxy group or the like on an aromatic ring, such as a phenoxy group, a tolyloxy group or a naphthyloxy group; an aralkyloxy group (preferably a C₇₋₁₈ aralkyloxy group) such as a benzyloxy group or a phenethyloxy group; an acyloxy group (preferably a C₁₋₁₂ acyloxy group) such as an acetyloxy group, a propionyloxy group, a (meth)acryloyloxy group, or a benzoyloxy group; a mercapto group; an alkylthio group (preferably a C₁₋₆ alkylthio group, and more preferably a C₁₋₄ alkylthio group) such as a methylthio group or an ethylthio group; an alkenylthio group (preferably a C₂₋₆ alkenylthio group, and more preferably a C₂₋₄ alkenylthio group) such as an allylthio group; an arylthio group (preferably a C₆₋₁₄ arylthio group) which may have a substituent of a C₁₋₄ alkyl group, a C₂₋₄ alkenyl group, a halogen atom, a C₁₋₄ alkoxy group or the like on an aromatic ring, such as a phenylthio group, a tolylthio group, or a naphthylthio group; an aralkylthio group (preferably a C₇₋₁₈ aralkylthio group) such as a benzylthio group or a phenethylthio group; a carboxy group; an alkoxycarbonyl group (preferably a C₁₋₆ alkoxycarbonyl group) such as a methoxycarbonyl group, an ethoxycarbonyl group, a propoxycarbonyl group or a butoxycarbonyl group; an aryloxycarbonyl group (preferably a C₆₋₁₄ aryloxycarbonyl group) such as phenoxycarbonyl group, a tolyloxycarbonyl group, or a naphthyloxycarbonyl group; an aralkyloxycarbonyl group (preferably a C₇₋₁₈ aralkyloxycarbonyl group) such as a benzyloxycarbonyl group; an amino group; a mono- or di-alkylamino group (preferably a mono- or di-C₁₋₆ alkylamino group) such as a methylamino group, an ethylamino group, a dimethylamino group or diethylamino group; an acylamino group (preferably a C₁₋₁₁ acylamino group) such as an acetylamino group, a propionylamino group, or a benzoylamino group; an oxetanyl group-containing group such as an ethyloxetanyloxy group; an acyl group such as an acetyl group, a propionyl group or a benzoyl group; an oxo group; and a group formed by bonding two or more of these via a C₁₋₆ alkylene group if necessary.

In formula (1) above, Y¹ and Y² are the same or different and represent a bivalent group represented by the following formula (a): 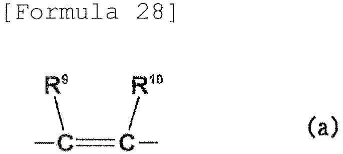 or a bivalent group represented by the following formula (b) :

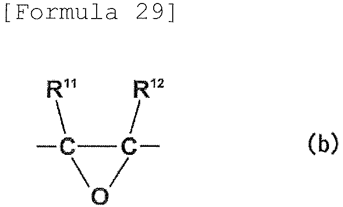

In formula (a) above, R⁹ and R¹⁰ are the same or different and represent a hydrogen atom, an alkenyl group which may have a substituent, an epoxidized alkenyl group which may have a substituent, or an alkyl group which may have a substituent. In formula (b) above, R¹¹ and R¹² are the same or different and represent a hydrogen atom, an alkenyl group which may have a substituent, an epoxidized alkenyl group which may have a substituent, or an alkyl group which may have a substituent. Examples of the alkenyl group which may have a substituent, the epoxidized alkenyl group which may have a substituent, and the alkyl group which may have a substituent used as R⁹ to R¹² include the same groups as those described above as the examples of R¹ to R⁸.

Incidentally, at least one of R¹ to R¹² in formula (1) above is an alkenyl group which may have a substituent, an epoxidized alkenyl group which may have a substituent, or an alkyl group which may have a substituent. In other words, a compound using a hydrogen atom as all of R¹ to R¹² in formula (1) is not embraced in the epoxy compound of the present invention.

The epoxy compound of the present invention is a compound having, in a molecule, at least one or more epoxy groups. In other words, when both Y¹ and Y² in formula (1) above are a bivalent group represented by formula (a), at least one of R¹ to R¹⁰ is an epoxidized alkenyl group which may have a substituent.

As the epoxy compound of the present invention, compounds of the following [i] to [iv] are particularly preferred.
[i] A compound represented by formula (1) above in which Y¹ and Y² both are a bivalent group represented by formula (b) (in particular, a bivalent group in which R¹¹ and R¹² both are a hydrogen atom).
[ii] A compound represented by formula (1) above in which at least one of (particularly, one of) R¹ to R⁴ is an epoxidized alkenyl group which may have a substituent (such as a glycidyl group or a 2-methyl glycidyl group) and at least one of (particularly, one of) R⁵ to R⁸ is an epoxidized alkenyl group which may have a substituent (such as a glycidyl group or a 2-methyl glycidyl group).
[iii] A compound represented by formula (1) above in which Y¹ and Y² both are a bivalent group represented by formula (b) (in particular, a bivalent group in which R¹¹ and R¹² both are a hydrogen atom), at least one of (particularly, one of) R¹ to R⁴ is an epoxidized alkenyl group which may have a substituent (such as a glycidyl group or a 2-methyl glycidyl group), and at least one of (particularly, one of) R⁵ to R⁸ is an epoxidized alkenyl group which may have a substituent (such as a glycidyl group or a 2-methyl glycidyl group).
[iv] A compound represented by formula (1) above in which Y¹ and Y² both are a bivalent group represented by formula (b) (in particular, a bivalent group in which R¹¹ and R¹² both are a hydrogen atom), at least one of (particularly, one of) R¹ to R⁴ is an alkyl group which may have a substituent (such as an alkyl group having 1 to 4 carbon atoms, and particularly a methyl group), and at least one of (particularly, one of) R⁵ to R⁸ is an alkyl group which may have a substituent (such as an alkyl group having 1 to 4 carbon atoms, and particularly a methyl group).

In particular, the compounds of [i] to [iv] above in which R in formula (1) is an alkylene group (a hexamethylene group, in particular), a bivalent group represented by formula (i) above (a xylylene group, in particular), a bivalent group represented by formula (ii) above (a phenylene-methylene-phenylene group, in particular), or a bivalent group represented by formula (iii) above (a bivalent group represented by formula (iii'), in particular) are preferred.

Specific examples of the epoxy compound of the present invention include compounds represented by the following formulas. Incidentally, R in the following formulas represents a bivalent group having one or more atoms as described above. Besides, X¹ and X² in the following formulas are the same or different and represent an alkylene group, an oxygen atom, a sulfur atom or no bond as described above. It is noted that corresponding various isomers (such as structural isomers and stereoisomers) are embraced in the following formulas.

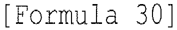

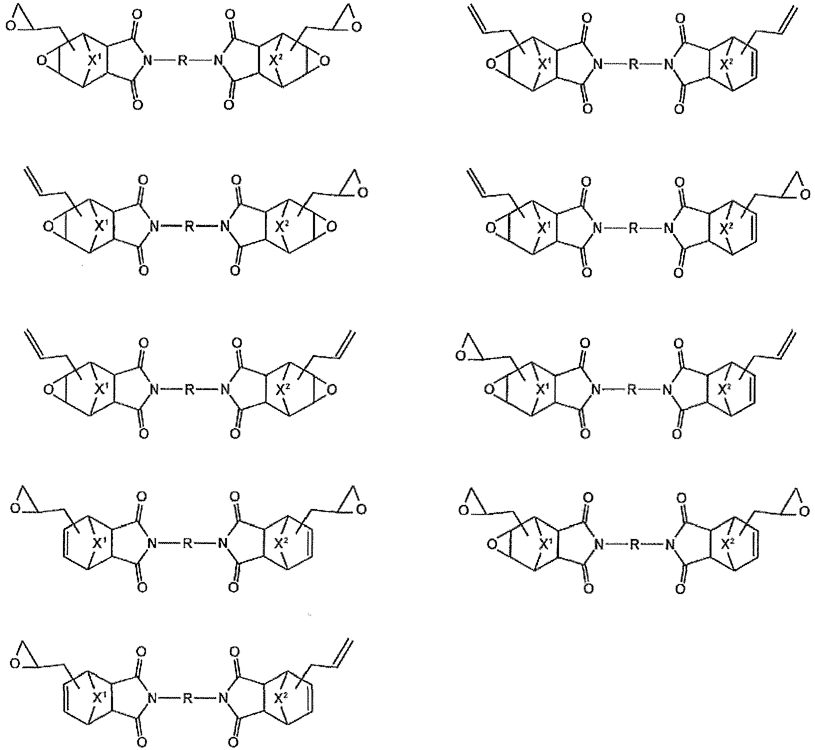

### [Production method for epoxy compound]

The epoxy compound of the present invention can be produced by a production method in which a compound represented by the following formula (2) 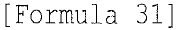 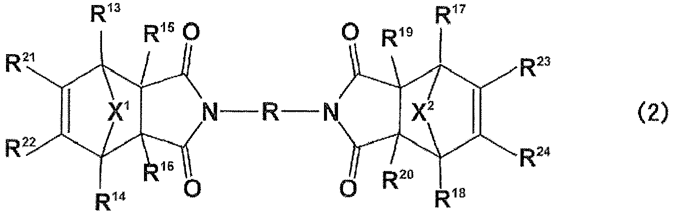 is reacted with an oxidant for producing the epoxy compound of the present invention (the compound represented by formula (1) above).

The compound represented by formula (2) above is a precursor of the epoxy compound of the present invention. In formula (2) above, X¹, X² and R are the same as those in formula (1) above. R¹³ to R²⁴ in formula (2) above are the same or different, and represent a hydrogen atom, an alkenyl group which may have a substituent, or an alkyl group which may have a substituent. Examples of the alkenyl group which may have a substituent and the alkyl group which may have a substituent used as R¹³ to R²⁴ include the same groups as those described above as the examples of R¹ to R⁸. It is noted that at least one of R¹³ to R²⁴ in formula (2) is an alkenyl group which may have a substituent or an alkyl group which may have a substituent.

The compound represented by formula (2) above can be obtained by reacting, for example, a compound (an acid anhydride) represented by the following formula (3): 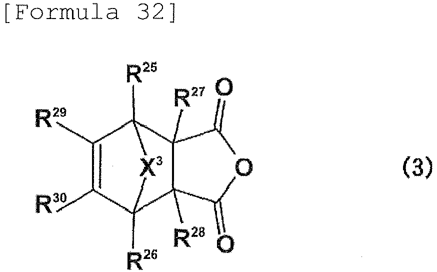 with a compound (diamine) represented by the following formula (4):
[Formula 33]

**H₂N-R-NH₂** (4)

X³ in formula (3) above represents the same groups as X¹ or X² in formula (2) above. R²⁵ to R³⁰ in formula (3) above represent the same groups as R¹³ to R²⁴ in formula (2) above, and represent a hydrogen atom, an alkenyl group which may have a substituent, or an alkyl group which may have a substituent. It is noted that at least one of R²⁵ to R³⁰ in formula (3) is an alkenyl group which may have a substituent or an alkyl group which may have a substituent.

R in formula (4) above represents the same group as R in formula (2) above (and formula (1) above), and represents a bivalent group having one or more atoms.

The reaction between the compound represented by formula (3) above and the compound represented by formula (4) above is allowed to proceed by a known and usual method. Specifically, the compound represented by formula (2) above can be produced by practicing the reaction in accordance with a method described in, for example, Japanese Patent Laid-Open No. 7-53516.

As the compound represented by formula (2) above, a commercially available product can be used, and for example, compounds available as a trade name "BANI-M", a trade name "BANI-X" and a trade name "BANI-H" (all manufactured by Maruzen Petrochemical Co., Ltd.) and the like can be used.

The reaction between the compound represented by formula (2) above and an oxidant is allowed to proceed in presence of a solvent or in the absence of a solvent. The solvent is not especially limited as long as it can homogeneously dissolve the compound represented by formula (2) above and the oxidant therein, and examples include: an alcohol such as t-butyl alcohol; an aliphatic hydrocarbon such as hexane, heptane or octane; an alicyclic hydrocarbon such as cyclohexane; an aromatic hydrocarbon such as benzene, toluene, xylene, or ethylbenzene; a halogenated hydrocarbon such as chloroform, methylene chloride, or 1,2-dichloroethane; an ester such as ethyl acetate; an amide such as N,N-dimethylformamide or N,N-dimethylacetamide; a nitrile such as acetonitrile, propionitrile or benzonitrile; a ketone such as acetone; and an organic acid such as acetic acid. Among these, a halogenated hydrocarbon and an ester (ethyl acetate in particular) are preferred as the solvent. Incidentally, one of these solvents can be singly used, or two or more of these can be used in combination.

The amount of use of the solvent is not especially limited but is preferably 100 to 2000 parts by weight, and more preferably 250 to 1000 parts by weight based on 100 parts by weight of the compound represented by formula (2) above.

The oxidant is not especially limited but a known and usual oxidant capable of epoxidizing a carbon-carbon unsaturated double bond (an ethylenic unsaturated bond) can be used, and examples include: an organic peroxide, such as hydrogen peroxide, performic acid, peracetic acid, trifluoroperacetic acid, perbenzoic acid, meta-chloroperbenzoic acid (mCPBA), or dimethyldioxirane; an oxo complex such as a salen complex; and a peroxide salt such as magnesium monoperoxyphthalate or oxone. Among these, meta-chloroperbenzoic acid is preferred from the viewpoint of small scale and handleability. On the other hand, hydrogen peroxide and peracetic acid are preferred from the viewpoint of simpleness of production processing. Besides, a reaction using peracetic acid is more preferred since the reaction can be performed in a uniform layer. The oxidant can be obtained by a known and usual method, and the production method therefor is not especially limited. For example, peracetic acid can be produced by air oxidation of acetaldehyde, and specifically, is produced by, for example, a method described in German Patent Laid-Open No. 1418465 or Japanese Patent Laid-Open No. 54-3006. When this method is employed, as compared with a case where an organic percarboxylic acid is synthesized from hydrogen peroxide for producing the organic percarboxylic acid through extraction with a solvent, a large amount of organic percarboxylic acid in a high concentration can be continuously synthesized, and hence, the percarboxylic acid can be substantially inexpensively obtained.

The amount of use of the oxidant is not especially limited but is preferably 0.1 to 4.0 equivalents, and more preferably 1.0 to 1.2 equivalents per equivalent of a carbon-carbon unsaturated double bond contained in the compound represented by formula (2) above. Incidentally, the epoxidation ratio of the compound represented by formula (2) above can be controlled by controlling the amount of use of the oxidant, so that various types of the epoxy compound of the present invention different in the epoxy equivalent can be thus produced.

The reaction between the compound represented by formula (2) above and the oxidant is allowed to proceed by, for example, adding the oxidant to a solution of the compound represented by formula (2) above and heating the resultant if necessary. The oxidant may be added at one time, or may be successively or continuously added.

The temperature in causing the reaction between the compound represented by formula (2) above and the oxidant (the reaction temperature) can be adjusted depending on the type of oxidant to be used or the like and is not especially limited, but is preferably -20 to 100°C, more preferably 0 to 70°C, and further more preferably 10 to 50°C. If the reaction temperature is lower than -20°C, the reaction speed and the progress of the reaction are insufficient, and hence, the yield of the epoxy compound represented by formula (1) may be lowered in some cases. On the other hand, if the reaction temperature exceeds 100°C, the oxidant and the product are easily decomposed, and hence, the yield of the epoxy compound represented by formula (1) may be lowered in some cases.

The time for causing the reaction between the compound represented by formula (2) above and the oxidant (the reaction time) is not especially limited, but is preferably 30 to 1440 minutes, more preferably 60 to 720 minutes, and further preferably 180 to 360 minutes. If the reaction time is shorter than 30 minutes, the progress of the reaction is insufficient, and hence, the yield of the epoxy compound represented by formula (1) may be lowered in some cases. On the other hand, if the reaction time exceeds 1440 minutes, the oxidant and the product are easily decomposed, and hence, the yield of the epoxy compound represented by formula (1) may be lowered in some cases.

Incidentally, the reaction between the compound represented by formula (2) above and the oxidant may be performed under a normal pressure, or may be performed under a reduced pressure or an increased pressure. The atmosphere employed in practicing the reaction is not especially limited as long as it does not inhibit the reaction, and for example, any of an air atmosphere, a nitrogen atmosphere, an argon atmosphere and the like may be employed. Besides, the reaction may be performed by any of a batch method, a semi-batch method, a continuous method and the like.

The reaction between the compound represented by formula (2) above and the oxidant can be terminated by, for example, adding a reductant such as sodium thiosulfate or sodium sulfite. Besides, an acid produced as a byproduct by the reaction can be neutralized by adding, to the reaction solution, an alkali such as sodium hydroxide, potassium hydroxide, sodium carbonate, potassium carbonate or sodium hydrogencarbonate.

After terminating the reaction, the compound represented by formula (1) obtained as a product can be separated and purified by, for example, separation means such as filtration, concentration, distillation, extraction, crystallization, recrystallization, or column chromatography, or separation means obtained by combining these.

The epoxy compound of the present invention has, in a molecule, one or more epoxy groups as shown in formula (1) above, and hence can form a cured product by reacting it with a known and usual epoxy curing agent such as an acid anhydride or polyamine, or by causing cationic polymerization to proceed, and the cured product exhibits excellent heat resistance derived from the imide skeleton contained in the epoxy compound of the present invention.

### [Curable epoxy resin composition]

A curable epoxy resin composition of the present invention is a curable composition containing the epoxy compound of the present invention as an essential component. Since the epoxy compound of the present invention is contained as an essential component, a cured product obtained by curing the curable epoxy resin composition exhibits excellent heat resistance. It is noted that the curable epoxy resin composition of the present invention may contain one of the epoxy compound of the present invention singly or may contain two or more kinds thereof.

The content of the epoxy compound of the present invention in the curable epoxy resin composition of the present invention is not especially limited but is preferably 1 to 99% by weight and more preferably 5 to 95% by weight based on the total amount (100% by weight) of epoxy compounds contained in the curable epoxy resin composition.

From the viewpoint of excellent handleability, the curable epoxy resin composition of the present invention is preferably in a liquid form at room temperature (for example, 25°C). More specifically, the viscosity of the curable epoxy resin composition of the present invention at 25°C is not especially limited but is preferably not more than 1,000,000 mPa·s (for example, 1 to 1,000,000 mPa·s), and more preferably not more than 100,000 mPa·s. If the viscosity at 25°C exceeds 1,000,000 mPa·s, the composition may be difficult to be handled in some cases. Incidentally, the viscosity at 25°C can be measured, for example, by using a digital viscometer (Model Number "DVU-EII", manufactured by Tokimekku Corp.) under the following conditions: Rotor: standard rotor of 1°34' x R24; temperature: 25°C; and rotor speed: 0.5 to 10 rpm.

The curable epoxy resin composition of the present invention preferably further contains an alicyclic epoxy compound that is in a liquid form at 25°C (hereinafter sometimes simply designated as the "alicyclic epoxy compound"). In this manner, even if the epoxy compound of the present invention is an epoxy compound that is in a solid form at 25°C, a curable epoxy resin composition that is in a liquid form at 25°C can be easily obtained without using a solvent or the like such as an organic solvent, and thus, the handleability can be improved. An example of the alicyclic epoxy compound includes a compound represented by the following formula (I):

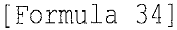

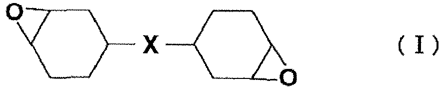

In formula (I) above, X represents a single bond or a linking group (a bivalent group having one or more atoms). Examples of the linking group include a bivalent hydrocarbon group, a carbonyl group, an ether bond, an ester bond, a carbonate group, an amide group, and a group obtained by linking a plurality of any of these.

The content of the alicyclic epoxy compound in the curable epoxy resin composition of the present invention is not especially limited but is preferably 1 to 99% by weight, and more preferably 5 to 95% by weight based on the total amount (100% by weight) of epoxy compounds contained in the curable epoxy resin composition.

The curable epoxy resin composition of the present invention may contain one of the above-described alicyclic epoxy compounds singly, or may contain two or more of these in combination. Incidentally, as the alicyclic epoxy compound, a commercially available product may be used, and examples include compounds available as a trade name "CELLOXIDE 2021P", a trade name "CELLOXIDE 2081", a trade name "CELLOXIDE 3000", a trade name "CELLOXIDE 2000", a trade name "EPOLEAD GT400", a trade name "CELVENUS B0084" and a trade name "CELVENUS B0177" (all manufactured by Daicel Corporation).

The curable epoxy resin composition of the present invention may contain another component (sometimes designated as the "additional component") in addition to the epoxy compound of the present invention and the alicyclic epoxy compound described above. Examples of the additional component include: an epoxy compound different from the epoxy compound of the present invention and the alicyclic epoxy compound described above; a component for curing the curable epoxy resin composition by reacting with the epoxy compound or by causing cation polymerization of the epoxy compound to proceed, such as a curing agent (of, for example, an acid anhydride or polyamine) or a curing catalyst; and a usual additive such as an antifoaming agent, a leveling agent,
a coupling agent, a surface active agent, an inorganic filler of silica, alumina or the like, a flame retardant, a coloring agent, an antioxidant, an ultraviolet absorber, an ion adsorbing material, a pigment, a release agent, or a phosphor.

### Examples

The present invention will now be described in more detail on the basis of Examples, and it is noted that the present invention is not limited to these Examples.

### Example 1

A nitrogen-purged flask having an inner capacity of 200 ml was charged with 6.5 g (0.013 mol) of a compound available as a trade name "BANI-X" (manufactured by Maruzen Petrochemical Co., Ltd., a compound represented by formula (2-1) below) and 32.5 ml of methylene chloride, and the compound was homogeneously dissolved under stirring. To the thus obtained solution, 13.75 g of meta-chloroperbenzoic acid (water-containing, having a purity of 70%, 4.4 equivalents, 0.056 mol) was added at 25°C over 15 minutes, and then a reaction was performed at 37°C for 7 hours. After confirming that the raw materials had disappeared by NMR, the resultant was cooled to room temperature, and the reaction was terminated by adding a 10% sodium thiosulfate aqueous solution (100 ml) to the reaction solution. This solution was transferred to a separating funnel to separate a water layer, and thereafter, a 10% sodium hydroxide aqueous solution (30 ml) was added to an organic layer to neutralize benzoic acid produced as a byproduct. This organic layer was washed with water twice to separate a water layer.

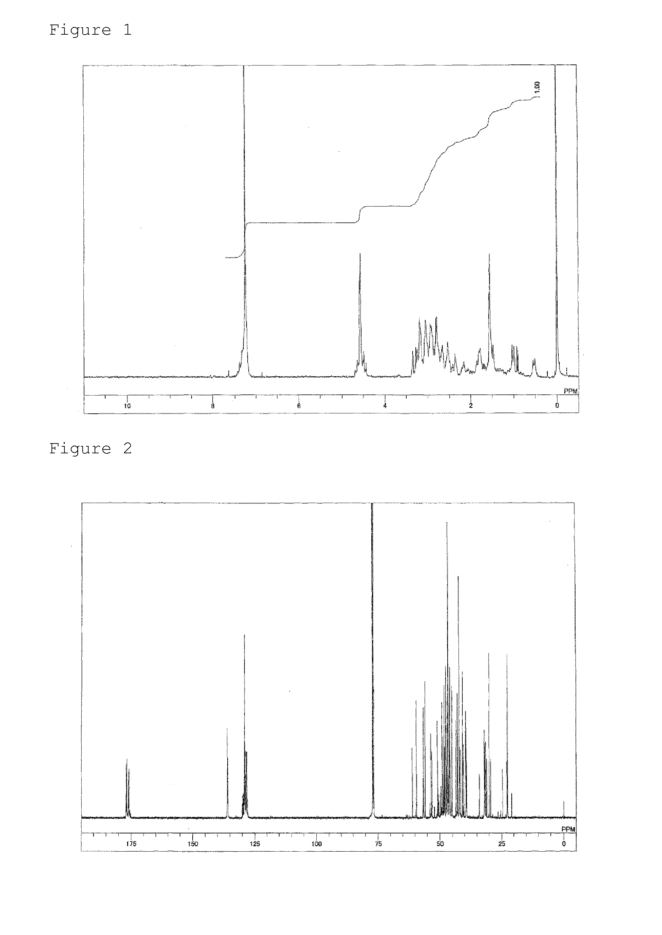

Thereafter, a low-boiling point component such as a solvent was removed by using an evaporator under conditions of 30°C, 3 mmHg and 30 minutes, and subsequently, the resultant was dried for 6 hours by using a vacuum dryer to obtain a product (7.2 g of an epoxy compound, yield 98%). As a result of the ¹H-NMR spectrum measurement (see Figure 1) and the ¹³C-NMR spectrum measurement (see Figure 2) of the thus obtained product, the obtained product was identified as a compound represented by formula (1-1) below. Incidentally, this identification was also supported on the basis of a fact that peaks of (M + 1) = 573 (m/z) and (M - 1) = 571 (m/z) were observed in the product while a peak of (M + 1) = 509 (m/z) was observed in the compound represented by formula (2-1) (BANI-X) used as the raw material.

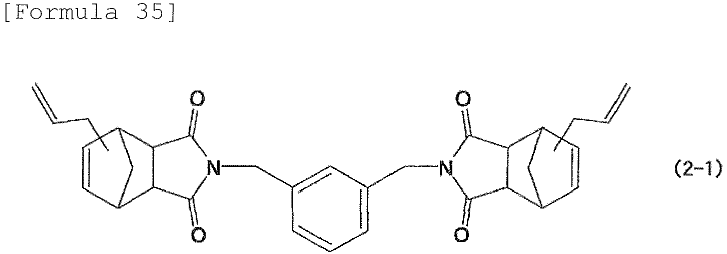

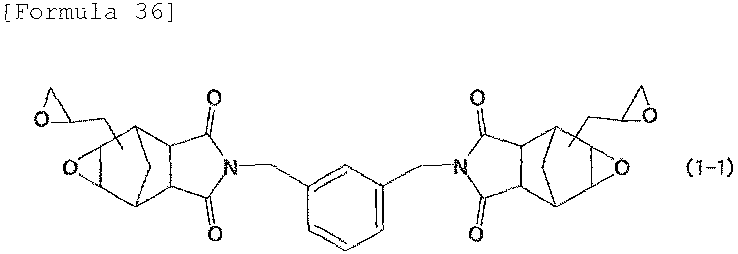

### Example 2

A nitrogen-purged flask having an inner capacity of 200 ml was charged with 20 g (0.039 mol) of the compound available as the trade name "BANI-X" (manufactured by Maruzen Petrochemical Co., Ltd., the compound represented by formula (2-1) above) and 110 ml of ethyl acetate, and the compound was homogeneously dissolved under stirring. To the thus obtained solution, 21.9 g of a 30% by weight peracetic acid solution in ethyl acetate was added at 27 to 37°C over 70 minutes, and then a reaction was performed at 37 to 53°C for 6 hours. After confirming that the raw materials had disappeared by the NMR, the resultant was cooled to room temperature, and the reaction was terminated by adding a 10% sodium hydroxide aqueous solution (75.7 ml) to the reaction solution. This solution was transferred to a separating funnel to separate a water layer, and thereafter, an organic layer was washed with water twice to separate a water layer.

Thereafter, a low-boiling point component such as a solvent was removed by using an evaporator under conditions of 30°C, 3 mmHg and 30 minutes, and subsequently, the resultant was dried at 40°C for 6 hours by using a vacuum dryer to obtain a product (16.0 g of an epoxy compound, yield 71%).

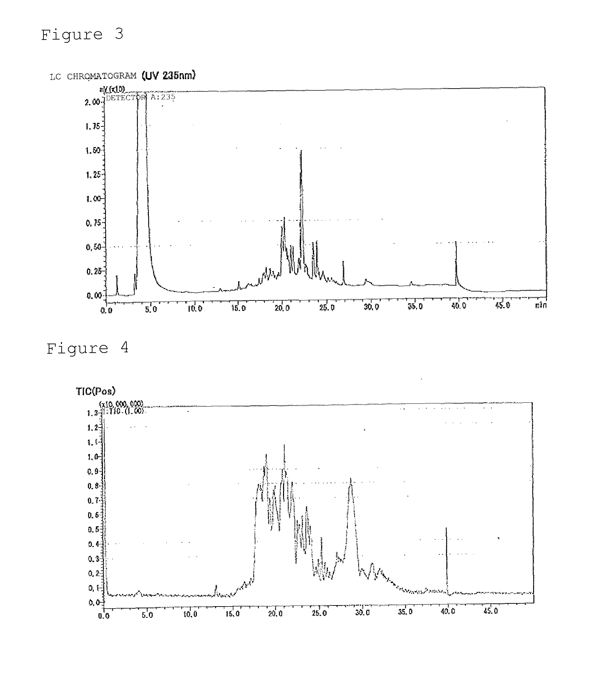

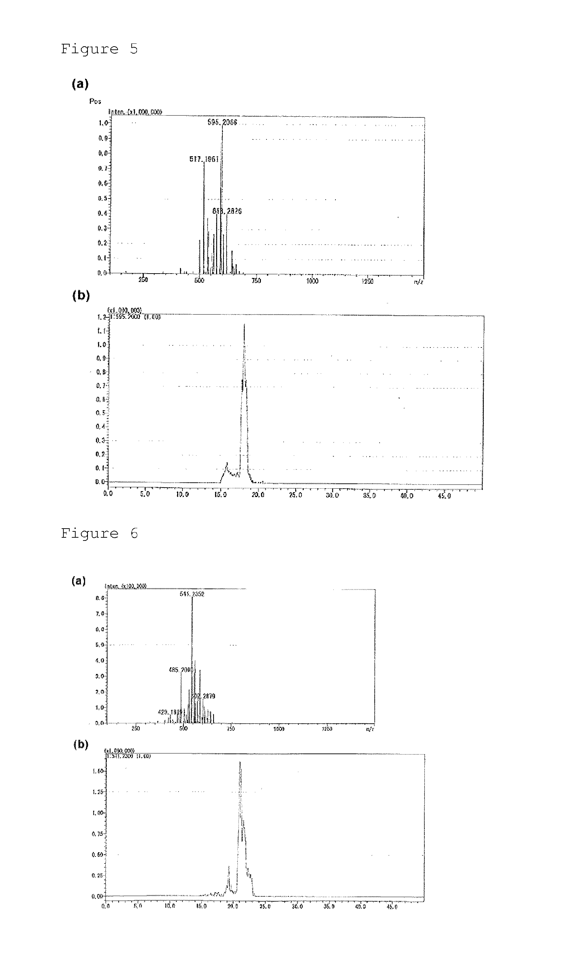

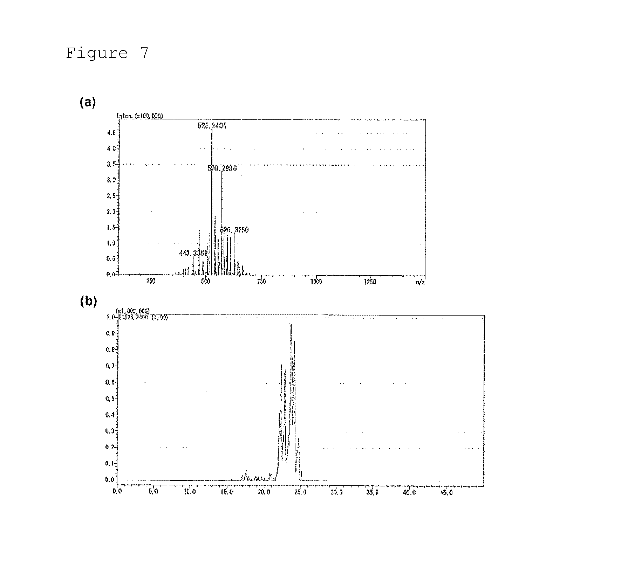

The product obtained as described above was subjected to LC-MS measurement. A UV chromatogram obtained by the LC-MS measurement is illustrated in Figure 3, and a total ion chromatogram is illustrated in Figure 4. Besides, a mass spectrum of a peak at the retention time of 15 to 20 minutes in the chromatogram obtained by the LC-MS measurement (the LC-MS chromatogram) is illustrated in Figure 5(a), and a mass chromatogram of a substance having m/z of 595 is illustrated in Figure 5(b). A mass spectrum of a peak at the retention time of 20 to 22 minutes in the LC-MS chromatogram is illustrated in Figure 6(a), and a mass chromatogram of a substance having m/z of 541 is illustrated in Figure 6(b). A mass spectrum of a peak at the retention time of 23 to 26 minutes in the LC-MS chromatogram is illustrated in Figure 7(a), and a mass chromatogram of a substance having m/z of 525 is illustrated in Figure 7(b). The substance having m/z of 595 of Figure 5(a) is regarded as a compound (having a molecular weight of 572) represented by formula (2-1) in which all of four carbon-carbon double bonds are epoxidized and to which a sodium ion is added. Besides, the substance having m/z of 541 of Figure 6(a) is regarded as a compound (having a molecular weight of 540) represented by formula (2-1) in which two of carbon-carbon double bonds are epoxidized and to which a proton is added. Furthermore, the substance having m/z of 525 of Figure 7(a) is regarded as a compound (having a molecular weight of 524) represented by formula (2-1) in which one of carbon-carbon double bonds is epoxidized and to which a proton is added. In this manner, it was confirmed by the LC-MS measurement that the compound obtained as described above includes at least the compounds represented by formula (2-1) in which one of, two of, or all four of the carbon-carbon double bonds are respectively epoxidized.

### Production Example 1

### (Production of bicyclohexyl-3,3'-diepoxide)

A reactor was charged with 406 g of bicyclohexy-3,3'-diene and 1217 g of ethyl acetate, and 457 g of a 30% by weight peracetic acid solution in ethyl acetate (having a water content of 0.41% by weight) was added thereto in a dropwise manner over approximately 3 hours while blowing nitrogen into a gas phase portion and while controlling the temperature within the reaction system at 37.5°C. After terminating the dropwise addition of the peracetic acid solution, the resultant was aged at 40°C for 1 hour to terminate the reaction. Besides, the crude solution obtained after terminating the reaction at 30°C was washed with water, and a low-boiling point compound was removed at 70°C and 20 mmHg, resulting in obtaining 415 g of a product (yield 85%, oxirane oxygen concentration: 14.7% by weight). The thus obtained product was identified as bicyclohexyl-3,3'-diepoxide by the ¹H-NMR spectrum measurement.

### Example 3

To 20 parts by weight of the epoxy compound obtained in Example 2 and 80 parts by weight of the bicyclohexyl-3,3'-epoxide (3,4,3',4'-diepoxy bicyclohexane) (epoxy equivalent of 103) obtained in Production Example 1, 227.1 parts by weight of a compound available as a trade name "RIKACID MH700F" (acid anhydride equivalent: 163) was added, and after melting and mixing the resultant at 50°C, 2.0 parts by weight of ethylene glycol and 0.5 part by weight of a compound available as a trade name "U-CAT 12XD" were added thereto, and the resultant was stirred at 25°C for 5 minutes to obtain a curable epoxy resin composition in a liquid form. Thereafter, the curable epoxy resin composition obtained as described above was poured into an aluminum cup, the temperature was increased to cure the composition by heating at 100°C for 2 hours, and subsequently at 150°C for 2 hours, and thus, a cured product (a cured product sample) was obtained.

### Example 4

To 50 parts by weight of the epoxy compound obtained in Example 2 and 50 parts by weight of the bicyclohexyl-3,3'-epoxide (3,4,3',4'-diepoxy bicyclohexane) (epoxy equivalent of 103) obtained in Production Example 1, 199.8 parts by weight of the compound available as the trade name "RIKACID MH700F" (acid anhydride equivalent: 163) was added, and after melting and mixing the resultant at 50°C, 2.0 parts by weight of ethylene glycol and 0.5 part by weight of the compound available as the trade name "U-CAT 12XD" were added thereto, and the resultant was stirred at 25°C for 5 minutes to obtain a curable epoxy resin composition in a liquid form. Thereafter, the curable epoxy resin composition obtained as described above was poured into an aluminum cup, the temperature was increased to cure the composition by heating at 100°C for 2 hours, and subsequently at 150°C for 2 hours, and thus, a cured product (a cured product sample) was obtained.

### Comparative Example 1

A curable epoxy resin composition and a cured product were obtained in the same manner as in Example 3 except that the composition of the curable epoxy resin composition was changed as shown in Table 1.

### • 5% Weight reduction temperature [Td (5%)] and 10% weight reduction temperature [Td (10%)]

By using a thermogravimetric analysis apparatus (TGA) ("EXSTAR 6000 TG/DTA 6200", manufactured by SII Nanotechnology Inc.), the 5% weight reduction temperature and the 10% weight reduction temperature of each cured product were measured in a nitrogen atmosphere under a condition of a temperature increasing rate of 10°C/min.

The compositions of the curable epoxy resin compositions and the evaluation results of the physical properties of the cured products are shown in Table 1.

[Table 1]

**(Table 1)**

| | | | | Comparative Example 1 | Example 3 | Example 4 |
|---|---|---|---|---|---|---|
| Curable epoxy resin composition | Epoxy compound | Bicyclohexyl-3,3'-diepoxide | Parts by weight | 100 | 80 | 50 |
| | | Epoxy compound obtained in Example 2 | Parts by weight | 0 | 20 | 50 |
| | Diluent | Ethylene glycol | Parts by weight | 2.0 | 2.0 | 2.0 |
| | Acid anhydride type curing agent | Rikacid MH700F | Parts by weight | 146.7 | 227.1 | 199.8 |
| | Catalyst | U-CAT 12XD | Parts by weight | 0.5 | 0.5 | 0.5 |
| Cured product | Heat resistance (TG/DTA) 30-400°C (10°C/min) | Td (5%) | °C | 332.1 | 338.6 | 344.5 |
| | | Td (10%) | °C | 343.3 | 346.5 | 351.5 |

### Industrial Applicability

A cured product obtained by curing an epoxy compound of the present invention or a curable epoxy resin composition containing the epoxy compound exhibits excellent heat resistance. Therefore, they can be used as highly heat-resistant epoxy resins.

## Claims

1. An epoxy compound represented by the following formula (1): 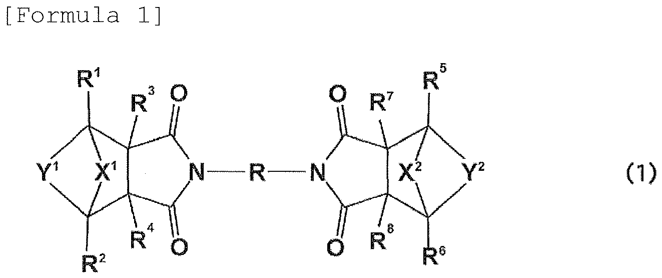 wherein X¹ and X² are the same or different and represent an alkylene group, an oxygen atom, a sulfur atom, or no bond; R represents a bivalent group having one or more atoms; R¹ to R⁸ are the same or different and represent a hydrogen atom, an alkenyl group which may have a substituent, an epoxidized alkenyl group which may have a substituent, or an alkyl group which may have a substituent; and Y¹ and Y² are the same or different and represent a bivalent group represented by the following formula (a): 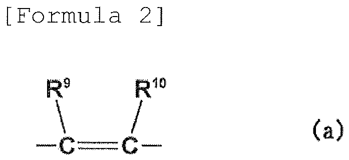 or a bivalent group represented by the following formula (b) : 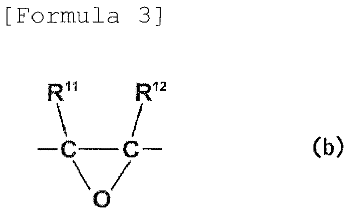 R⁹ and R¹⁰ in formula (a) are the same or different and represent a hydrogen atom, an alkenyl group which may have a substituent, an epoxidized alkenyl group which may have a substituent, or an alkyl group which may have a substituent; and R¹¹ and R¹² in formula (b) are the same or different and represent a hydrogen atom, an alkenyl group which may have a substituent, an epoxidized alkenyl group which may have a substituent, or an alkyl group which may have a substituent; provided that at least one of R¹ to R¹² in formula (1) is an alkenyl group which may have a substituent, an epoxidized alkenyl group which may have a substituent, or an alkyl group which may have a substituent; and when both Y¹ and Y² in formula (1) are a bivalent group represented by formula (a), at least one of R¹ to R¹⁰ is an epoxidized alkenyl group which may have a substituent.

2. A production method for an epoxy compound,
wherein a compound represented by the following formula (2): 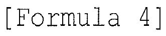 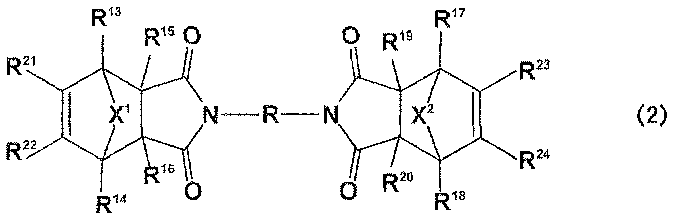 wherein X¹ and X² are the same or different and represent an alkylene group, an oxygen atom, a sulfur atom or no bond; R represents a bivalent group having one or more atoms; R¹³ to R²⁴ are the same or different and represent a hydrogen atom, an alkenyl group which may have a substituent, or an alkyl group which may have a substituent; provided that at least one of R¹³ to R²⁴ in formula (2) is an alkenyl group which may have a substituent or an alkyl group which may have a substituent,
is reacted with an oxidant to produce an epoxy compound represented by the following formula (1): 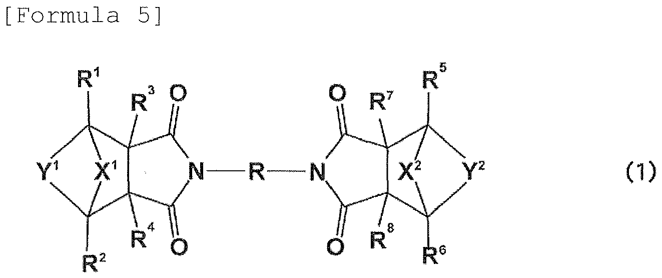 wherein X¹, X² and R are the same as those defined above; R¹ to R⁸ are the same or different and represent a hydrogen atom, an alkenyl group which may have a substituent, an epoxidized alkenyl group which may have a substituent, or an alkyl group which may have a substituent; and Y¹ and Y² are the same or different and represent a bivalent group represented by the following formula (a): 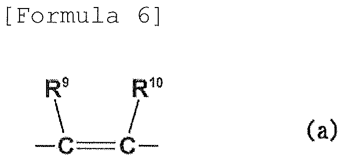 or a bivalent group represented by the following formula (b) : 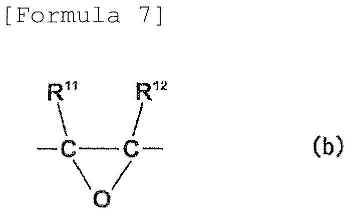 R⁹ and R¹⁰ in formula (a) are the same or different and represent a hydrogen atom, an alkenyl group which may have a substituent, an epoxidized alkenyl group which may have a substituent, or an alkyl group which may have a substituent; and R¹¹ and R¹² in formula (b) are the same or different and represent a hydrogen atom, an alkenyl group which may have a substituent, an epoxidized alkenyl group which may have a substituent, or an alkyl group which may have a substituent; provided that at least one of R¹ to R¹² in formula (1) is an alkenyl group which may have a substituent, an epoxidized alkenyl group which may have a substituent, or an alkyl group which may have a substituent; and when both Y¹ and Y² in formula (1) are a bivalent group represented by formula (a), at least one of R¹ to R¹⁰ is an epoxidized alkenyl group which may have a substituent.

3. A curable epoxy resin composition comprising the epoxy compound according to claim 1.

4. The curable epoxy resin composition according to claim 3, which is in a liquid form at 25°C.

5. The curable epoxy resin composition according to claim 3 or 4, further comprising an alicyclic epoxy compound that is in a liquid form at 25°C.

6. A cured product obtained by curing the curable epoxy resin composition according to any one of claims 3 to 5.
